# EUROPEAN PATENT SPECIFICATION

(11) **EP 4 519 244 B1**
(45) Date of publication and mention of the grant of the patent: **31.12.2025**
(21) Application number: 22727791.0
(22) Date of filing: 03.05.2022
(51) Int. Cl.: C07C 255/46, C11B 9/00

(54) **METHYL AND NITRILE SUBSTITUTED CYCLOHEPTANE DERIVATIVES FOR USE AS A FRAGRANCE**
METHYL UND NITRIL SUBSTITUIERTE CYCLOHEPTANE ZUR VERWENDUNG ALS DUFTSTOFFE
CYCLOHEPTANES SUBSTITUÉS PAR UN MÉTHYLE ET UN NITRILE POUR UN USAGE EN TANT QUE PARFUM

(43) Date of publication of application: 12.03.2025
(73) Proprietor: Symrise AG, 37603 Holzminden (DE)
(72) Inventor: HÖLSCHER, Bernd, 37620 Halle (DE); CHANDRASEKARAN, Vijayanand, 37603 Holzminden (DE)
(74) Representative: Siegel, Sven
(86) International application number: PCT/EP2022/061833
(87) International publication number: WO 2023/213381

(56) References cited:
- EP-A2- 0 164 763
- DE-A1- 2 061 517
- KLÄRNER FRANK-GERRIT ET AL: "Zur Stereochemie der Norcaradien-Norcaradien-Umlagerung, II. Die thermische Umlagerung optisch aktiver [Norcaradien <-> Cycloheptatrien]-Derivate", CHEMISCHE BERICHTE, vol. 112, no. 4, 1 April 1979 (1979-04-01), DE, pages 1168 - 1188, XP055981948, ISSN: 0009-2940, Retrieved from the Internet <URL:https://onlinelibrary.wiley.com/doi/pdf/10.1002/cber.19791120412> DOI: 10.1002/cber.19791120412

## Description

### FIELD OF THE INVENTION

The present invention relates to compounds of Formula I, their use as a fragrance as well as to their production method. Furthermore, the present invention relates to fragrance compositions, their use and perfumed products.

### BACKGROUND OF THE INVENTION

Despite a large number of existing fragrances, the perfume industry also continues to have a general need for new fragrances which have additional properties beyond their primary, namely olfactory, properties possess positive secondary properties, such as higher stability under certain application conditions, a higher substantivity and/or diffusivity or better adhesion, or through synergy effects with other fragrances lead to better sensory profiles.

Fragrances that are characterized by the above-mentioned positive secondary properties allow an increased effectiveness in the production of fragrance compositions and perfumed products. So e.g., through the use of fragrances with a better sensory profile, a higher substantivity and/or better adhesion, the number and the amounts of fragrances used in corresponding formulations can be optimized and / or minimized, resulting in a sustainable conservation of resources in the manufacture of perfumed products.

There is therefore a need in the perfume industry in particular for further fragrances with improved sensory profiles and/or secondary properties.

Thus, the underlying problem of the present invention was to provide new compounds, in particular new fragrances with the additional ability to enhance positive and beneficial fragrance aspects of other fragrances. In particular, the new fragrance should also be characterized by improving the stability, solubility and overall performance of other fragrances, as well as reducing the required dosage. Furthermore, the new fragrance should be renewable as well as stable. Finally, the fragrance compositions themselves should have excellent biodegradability and be harmless to humans and the environment.

Compounds having some structural resemblance for a use in perfumery are disclosed in DE 20 61 517 and EP 0 164 763. Reactivity studies with an unsaturated analog of the claimed compound are disclosed in F.G. Klärner et al. Chem. Ber. 1979, vol. 112, pages 1168-1188.

### DESCRIPTION OF THE INVENTION

This is solved by at least one compound of Formula I 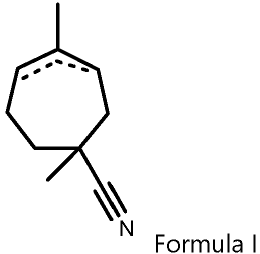 wherein each of the dotted lines represents a carbon-carbon single bond or a carbon-carbon double bond.

The compounds according to Formula I to be used according to the invention may be present in any stereoisomeric form or may be present as any mixture of stereoisomers.

What has been said herein for compounds of Formula I, in particular the advantages described herein, also apply to a mixture of stereoisomers of compounds of Formula I or to a mixture of different compounds of Formula I.

For the sake of clarity, by the expression "the dotted lines represent a carbon-carbon single bond or a carbon-carbon double bond", or the similar, it is meant the normal meaning understood by a person skilled in the art, i.e. that the whole bonding (solid and dotted line) between the carbon atoms connected by said dotted line is a single or a double bond.

In a preferred embodiment according to the invention, the at least one compound of Formula I is 1,4-dimethylcyclohept-3-ene-1-carbonitrile according to Formula II: 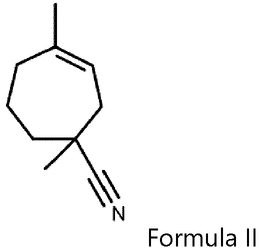 Preferably, 1,4-dimethylcyclohept-3-ene-1-carbonitrile has a molecular mass of 149.2, a molecular formula of C₁₀H₁₅N and has at least one of the following odor characteristics: coconut, green, fatty.

In a preferred embodiment according to the invention, the at least one compound of Formula I is 1,4-dimethylcyclohept-4-ene-1-carbonitrile according to Formula III: 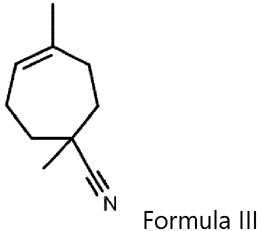 Preferably, 1,4-dimethylcyclohept-4-ene-1-carbonitrile has a molecular mass of 149.2, a molecular formula of C₁₀H₁₅N and has at least one of the following odor characteristics: coconut, green, fatty.

The present invention further relates to a mixture comprising or consisting of at least two compounds according to Formula I. Preferably, the at least two compounds according to Formula I are selected from
(a) 1,4-dimethylcyclohept-3-ene-1-carbonitrile 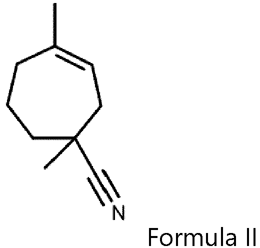 and
(b) 1,4-dimethylcyclohept-4-ene-1-carbonitrile

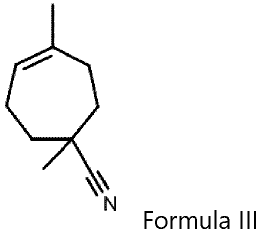

Preferably, the ratio of 1,4-dimethylcyclohept-3-ene-1-carbonitrile and 1,4-dimethylcyclohept-4-ene-1-carbonitrile in the mixture is from 5:2 to 2:5. Most preferred the ratio of 1,4-dimethylcyclohept-3-ene-1-carbonitrile and 1,4-dimethylcyclohept-4-ene-1-carbonitrile in the mixture is 3:2.

The compounds of Formula I possess olfactory properties which are quite unique and which clearly differ from and also surpass those of known odoriferous substances. Therefore, one aspect of the present invention relates to the use of a compound according to Formula I as a fragrance, 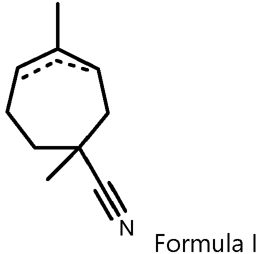 wherein each of the dotted lines represents a carbon-carbon single bond or a carbon-carbon double bond.

The suitability of the compounds of Formula I as fragrances was previously unknown. It is therefore particularly surprising that in the already well-studied field a fragrance with valuable, interesting and complex olfactory properties could be found.

As mentioned above, the suitability as a fragrance for the compounds of Formula I is not known. Likewise, no precise odor description is available, however, according to the inventors, the compounds of Formula I smell in particular coconut, green and/or fatty. Consequently, the inventors have made the surprising discovery that the compounds of Formula I are suitable as a fragrance and, in small dosages, produce special effects in combination with other fragrances which are not necessarily related to their own odor but, in combination with other, especially floral fragrances, convey a completely different note.

In a preferred embodiment according to the invention, the compound of Formula I is selected from 1,4-dimethylcyclohept-3-ene-1-carbonitrile and/or 1,4-dimethylcyclohept-4-ene-1-carbonitrile

In a preferred embodiment according to the invention, the compounds of Formula I are used to obtain at least one of the following fragrance notes: coconut and/or green.

Beyond the primary, namely olfactory, properties, the compounds of Formula I additionally possess positive secondary properties, in particular a high substantivity compared to fragrances with similar olfactory properties, as well as a high stability in certain media and preparations, a high extensibility, and is also biodegradable.

Moreover, the compounds of Formula I to be used according to the invention can enhance the intensity of a fragrance mixture (fragrance composition). The compounds described herein can therefore be used to impart roundness, naturalness, more volume, clearness, freshness, strength and/or fruitiness to a fragrance composition.

In a preferred embodiment according to the invention, the compound or the compounds of Formula I is/are used in a fragrance composition in an amount of 0.01 to 50% by weight, based on the total weight of the fragrance composition. More preferably, the compound or the compounds of Formula I is/are used in a fragrance composition in an amount of 0.01 to 10% by weight, based on the total weight of the fragrance composition.

In a further preferred embodiment according to the invention, the at least one compound of Formula I is used with one or more further fragrances, preferably within a fragrance composition. Preferably, the weight ratio of the at least one compound of Formula I to the total amount of other fragrances in the fragrance composition is in the range of 1:1000 to 1:0.1. More preferably, the weight ratio of the at least one compound of Formula I to the total amount of other fragrances in the fragrance composition is in the range of 1:1000 to 1:10.

The compounds of Formula I can be used in a variety of products; they can be used as a single fragrance, but they can be combined particularly advantageously with other fragrances in different proportions to form fragrance compositions, and novel and original perfume compositions can also be created. Accordingly, one aspect of the invention also relates to a fragrance composition and possibly further constituents (solvents or the like), which contains at least one compound of Formula I.

The fragrance composition according to the invention comprises or consists of at least one compound of Formula I 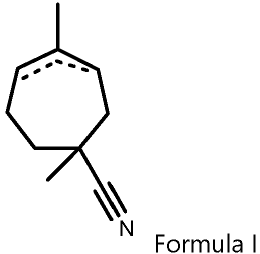 wherein each of the dotted lines represents a carbon-carbon single bond or a carbon-carbon double bond; and at least one further fragrance which is no compound of Formula I.

Preferably, the fragrance composition according to the invention comprises or consists of at least one compound of Formula I and two or more further fragrances. More preferably, the fragrance composition according to the invention comprises or consists of at least one compound of Formula I and three or more further fragrances. Most preferably, the fragrance composition according to the invention comprises or consists of at least one compound of Formula I and four or more further fragrances.

The compounds according to Formula I are usually used in a sensory effective amount, i.e. in a total amount in which it exerts a sensory effect.

Preferably, the weight ratio of the at least one compound of Formula I to the total amount of other fragrances is in the range of 1:1000 to 1:0.1, preferably from 1:1000 to 1:10.

In this context, it is preferred if the total amount the at least one compound of Formula I is in the range from 0.0001 to 99.9% by weight, preferably from 0.001 to 99.5% by weight, particularly preferably from 0.01 to 99% by weight, from 0.01 to 90% by weight, from 0.01 to 50% by weight, from 0.01 to 20% by weight and particularly preferably from 0.01 to 10% by weight, in particular from 0.05 to 1% by weight, in each case based on the total weight of the composition.

In a preferred embodiment according to the invention, at least one compound of Formula I is contained in the fragrance composition in a sensory effective amount sufficient to modify an existing fragrance to make it rounder, more natural, more volume, clearer, fresher, stronger and/or fruitier.

Examples of fragrances which may advantageously be combined with compounds of Formula I in the context of the present invention can be found, for example, in S. Arctander, Perfume and Flavor Materials, Vol. I and II, Montclair, N. J. 1969, self-published, or K. Bauer ei a/., Common Fragrance and Flavor Materials, 4th Edition, Wiley-VCH, Weinheim 2001.

Specifically mentioned are: Extracts from natural raw materials such as essential oils, concretes, absolutes, resins, resinoids, balsams, tinctures such as.

Ambergris tincture; Amyris oil; Angelica seed oil; Angelica root oil; Anise oil; Valerian oil; Basil oil; tree moss absolute; bay oil; mugwort oil; benzoeresin; bergamot oil; Beeswax absolue; Birch tar oil; Bitter almond oil; Savory oil; Bucco leaf oil; Cabreuva oil; Cade oil; Calmus oil; Camphor oil; Cananga oil; Cardamom oil; Cascarilla oil; Cassia oil; Cassie-absolue; Castoreum-absolue; Cedar leaf oil; Cedarwood oil; Cistus oil; Citronella oil; Citron oil; Copaiva balsam; Copaiva balsam oil; Coriander oil; Costus root oil; Cumin oil; Cypress oil; Davana oil; Dill herb oil; Dill seed oil; Eau de brouts-Absolue; Oak moss absolute; Elemi oil; Tarragon oil; Eucalyptus citriodora oil; Eucalyptus oil; Fennel oil; Spruce needle oil; Galbanum oil; Galbanum resin; Geranium oil; Grapefruit oil; Guaiac wood oil; Gurjun balsam; Gurjun balsam oil; Helichrysum absolute; Helichrysum oil; Ginger oil; Iris root absolute; Iris root oil; Jasmine absolute; Calamus oil; Chamomile oil blue; Chamomile oil roman; Carrot seed oil; Cascarilla oil; Pine needle oil; Spearmint oil; Caraway oil; labdanum oil; labdanum absolute; labdanum resin; lavandin absolute; Lavandin oil; Lavender absolute; Lavender oil; Lemongrass oil; Lovage oil; Lime oil distilled; Lime oil pressed; Linaloe oil; Litsea cubeba oil; Bay leaf oil; Mace oil; Marjoram oil; Mandarin oil; Massoir bark oil; Mimosa absolute; Musk grain oil; Musk tincture; Muscat sage oil; Nutmeg oil; Myrrh absolute; Myrrh oil; Myrtle oil; Clove leaf oil; Clove flower oil; Neroli oil; Olibanum absolute; Olibanum oil; Opopanax oil; orange blossom absolute; orange oil; origanum oil; palmarosa oil; patchouli oil; Perilla oil; Perubalsam oil; Parsley leaf oil; Parsley seed oil; Petitgrain oil; Peppermint oil; Pepper oil; Allspice oil; Pine oil; Poley oil; Rose absolute; Rosewood oil; Rose oil; Rosemary oil; Sage oil Dalmatian; Sage oil Spanish; Sandalwood oil; Celery seed oil; Spicy lavender oil; Star anise oil; Styrax oil; Tagetes oil; Fir needle oil; Tea tree oil; Turpentine oil; Thyme oil; Tolu balsam; Tonka absolue; tuberose-absolue; vanilla extract; violet leaf-absolue; verbena oil; vetiver oil; juniper berry oil; wine yeast oil; wormwood oil; wintergreen oil; ylang oil; hyssop oil; civet-absolue; cinnamon leaf oil; cinnamon bark oil and fractions thereof, or Ingredients isolated therefrom;

Individual odorants from the group of hydrocarbons, such as 3-carene; α-pinene; β-pinene; a-terpinene; γ-terpinene; p-cymene; bisabolene; camphene; caryophyllene; cedrene; farnesene; limonene; longifolene; myrcene; ocimene; valencene; (E,Z)-1 ,3,5-undecatriene; styrene; diphenylmethane;
of the aliphatic alcohols such as Hexanol; octanol; 3-octanol; 2,6-dimethylheptanol; 2-methyl-2-heptanol; 2-methyl-2-octanol; (E)-2-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
of aliphatic aldehydes and their acetals, such as Hexanal; heptanal; octanal; nonanal; decanal; undecanal; dodecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6, 10-trimethyl-9-undecenal; 2,6, 10-trimethyl-5,9-undecadienal; heptanaldiethyl acetal; 1 , 1-dimethoxy-2,2,5-trimethyl-4-hexene; citronellyloxyacetaldehyde; 1 -( 1 -methoxy-propoxy)-(E/Z)-3-hexene;
of aliphatic ketones and their oximes such as 2-heptanone; 2-octanone; 3-octanone; 2-nonanone; 5-methyl-3-heptanone; 5-methyl-3-heptanone oxime; 2,4,4,7-tetramethyl-6-octen-3-oη; 6-methyl-5-hepten-2-one;
of aliphatic sulfur-containing compounds such as 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthen-8-thiol;
of aliphatic nitriles such as 2-nonenoic acid nitrile; 2-undecenoic acid nitrile; 2-tridecenoic acid nitrile; 3, 12-tridecadienoic acid nitrile; 3,7-dimethyl-2,6-octadienoic acid nitrile; 3,7-dimethyl-6-octenoic acid nitrile;
esters of aliphatic carboxylic acids, e.g. (E)- and (Z)-3-hexenyl formate; ethyl acetoacetate; isoamyl acetate; 3,5,5-trimethylhexyl acetate; 3-methyl-2-butenyl acetate; (E)-2-hexenyl acetate; (E)- and (Z)-3-hexenyl acetate; Octyl acetate; 3-octyl acetate; 1-octene-3-yl acetate; ethyl butyrate; butyl butyrate; isoamyl butyrate; hexyl butyrate; (E)- and (Z)-3-hexenyl isobutyrate; Hexyl crotonate; ethyl isovalerate; ethyl 2-methylpentanoate; ethyl hexanoate; allyl hexanoate; ethyl heptanoate; allyl heptanoate; ethyl octanoate; Ethyl (E,Z)-2,4-decadienoate; methyl 2-octinate; methyl 2-noninate; allyl 2-isoamyloxyacetate; M ethy 1-3,7-dimethyl-2,6-octadiene-oate;4-methyl-2-pentyl crotonate;
of acyclic terpene alcohols such as. Geraniol; nerol; lavadulol; nerolidol; farnesol; tetrahydrolinalool; tetrahydrogeraniol; 2,2-Dimethyl-3-(3-methylphenyl)propan-1-ol; 2,6-dimethyl-7-octen-2-ol; 2,6-dimethyloctan-2-ol; 2- methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-Dimethyloct-6-en-1-ol; (2E)-3,7-Dimethylocta-2,6-dien-1-ol; 3,7-dimethyl-1 ,5,7-octatrien-3-ol 2,6-dimethyl-2,5J-octatrien-1-ol; and their formates, acetates, propionates, isobutyrates, butyrates, isovalerianates, pentanoates, hexanoates, crotonates, tiglinates and 3-methyl-2-butenoates;
of the acyclic terpene aldehydes and ketones such as citronellal; 7-methoxy-3,7-dimethyloctanal; 2,6,10-trimethyl-9-undecenal; geranylacetone; and the dimethyl and diethylacetals of geranial, neral,
of cyclic terpene alcohols such as. menthol; isopulegol; alpha-terpineol; terpinenol-4; menthan-8-ol; menthan-1-ol; menthan-7-ol; borneol; isoborneol; linalool oxide; nopol; cedrol; ambrinol; vetiverol; guaiaol; and their formates, acetates, propionates, isobutyrates, butyrates, isovalerianates, pentanoates, hexanoates, crotonates, tiglinates and 3-methyl-2-butenoates;
of cyclic terpene aldehydes and ketones such as Menthone; isomenthone; 8-mercaptomenthan-3-oη; carvone; camphor; fenchone; alpha-lonone; beta-lonone; alpha-n-methylionone; beta-n-methylionone; alpha-Isomethylionone; beta-Isomethylionone; alpha-irone; beta-damascenone; 1-(2,4,4-trimethyl-2-cyclohexen-1-yl)-2-buten-1-one; 1 ,3,4,6,7,8a-Hexahydro-1 , 1 ,5,5-tetramethyl-2H-2,4a-methano-naphthalen-8(5H)-on;2-Methyl-4-(2,6,6-trimethyl-1-cyclohexen-1-yl)-2-butenal; Nootkatone; dihydronootkatone; 4,6,8-megastigmatriene-3-on; alpha-sinensal; beta-sinensal; acetylated cedarwood oil (methylcedrylketone);
of cyclic alcohols such as 4-tert-butylcyclohexanol; 3,3,5-trimethylcyclohexanol; 3-Isocamphylcyclohexanol; 2,6,9-trimethyl-Z2,Z5,E9-cyclododecatrien-1-ol; 2-Isobutyl-4-m ethyltetrahyd ro-2H-pyran-4-ol; 4-cyclohexylbutan-2-ol;
of cycloaliphatic alcohols such as. alpha,3,3-trimethylcyclohexylmethanol; 1-(4-Isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
cyclic and cycloaliphatic ethers, e.g. Cineol; cedryl methyl ether; cyclododecyl methyl ether;1 , 1-dimethoxycyclododecane; (ethoxymethoxy)cyclo-dodecane; alpha-cedrene epoxide; 3a,6,6,9a-tetramethyldodecahydro-naphtho[2, 1-b]furan; 3a-ethyl-6,6,9a-trimethyldodecahydronaphtho[2, 1 b]furan; 1 ,5,9-tri-methyl-13-oxabicyclo[10. 1 .0]trideca-4,8-diene; rose oxide; 2-(2,4-dimethyl-3-cyclohexen-1-yl)-5-m ethyl-5-( 1 - methyl propyl)- 1 , 3-d ioxane;
of cyclic and macrocyclic ketones such as 4-tert.- butylcyclohexanone ; 2,2,5-trimethyl-5-pentylcyclopentanone ; 2-heptylcyclopentanone ; 2-pentyl-cyclopentanone ; 2-hyd roxy-3-m ethyl-2-cyclopenten- 1 -one; 3-methyl-cis-2-penten- 1 -yl-2-cyclopenten- 1 -one ; 3-methyl-2-pentyl-2-cyclopenten-1-one ; 3-methyl-4-cyclopentadecenone ; 3-methyl-5-cyclopentadecenone ; 3-methylcyclopenta-decanone ; 4-(1-ethoxyvinyl)-3, 3,5,5-tetramethylcyclohexanone ; 4-tert. - pentylcyclo-hexanone; 5-cyclohexadecen-1-one; 6,7-dihydro-1 , 1 ,2,3,3-pentamethyl-4(5H)-indanone; 8-cyclohexadecen-1-one; 9-cycloheptadecen-1-oη; cyclopentadecanone; cyclohexadecanone;
of cycloaliphatic aldehydes such as 2-methyl-4-(2,2,6-trimethyl-cyclohexen-1-yl)-2-butenal; 4-(4-hydroxy-4-methylpentyl)-3-cyclohexenecarbaldehyde; 4-(4-methyl-3-penten-1-yl )-3-cyclohexenecarbald ehyde ;
of cycloaliphatic ketones, e.g. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2, 3,8,8-tetramethyl-1 , 2,3,4,5,6,7, 8-octahydro-2-naphthalenyl methyl ketone; methyl 2,6, 10-trimethyl-2,5,9-cyclododecatrienyl ketone; tert. - butyl-(2,4-dimethyl-3-cyclohexen-1-yl)ketone;
of ester of cyclic alcohols such as 2-tert-butylcyclohexyl acetate; 4-tert-butylcyclohexyl acetate; 2-tert-pentylcyclohexyl acetate; 4-tert-pentylcyclohexyl acetate; 3,3, 5-tri methylcyclohexyl acetate; decahyd ro-2-naphthyl acetate; 2-cyclo-pentylcyclopentyl crotonate; 3-pentyl tetrahydro-2H-pyran-4-yl acetate; decahydro-2,5,5,8a-tetramethyl-2-naphthyl acetate; 4,7-methano-3a,4,5,6,7,7a-hexa-hydro-5, resp. 6-indenyl acetate; 4,7-methano-3a,4,5,6,7,7a-hexahydro-5, or 6-indenyl propionate; 4,7-methano-3a,4,5,6,7,7a-hexahydro-5, or 6-indenyl isobutyrate; 4,7-methanooctahydro-5, or 6-indenyl acetate;
of esters of cycloaliphatic alcohols such as 1-cyclohexylethyl crotonate;
of esters of cycloaliphatic carboxylic acids, e.g. Allyl 3-cyclohexyl propionate; allyl cyclohexyloxy acetate; ice and trans-methyl dihydrojasmonate; ice and trans-methyl jasmonate; methyl 2-hexyl-3-oxocyclopentane carboxylate; ethyl 2-ethyl-6,6-dimethyl-2-cyclohexene carboxylate; ethyl 2,3,6,6-tetramethyl-2-cyclohexene carboxylate; ethyl 2-methyl-1 ,3-dioxolane-2-acetate;
of araliphatic alcohols such as Benzyl alcohol; 2-phenylethanol; 1-phenylethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1 , 1-dimethyl-2-phenylethyl alcohol; 1 , 1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-Isopropylphenyl)ethanol;
of esters of araliphatic alcohols and aliphatic carboxylic acids, e.g. Benzyl acetate; benzyl propionate; benzyl isobutyrate; benzyl isovalerate; 2-phenylethyl acetate; 2-phenylethyl propionate; 2-phenylethyl isobutyrate; 2-phenyl ethyl isovalerate; 1-phenylethyl acetate; alpha-trichloromethyl benzyl acetate; alpha, alpha-dimethylphenyl ethyl acetate; alpha,alpha-dimethylphenyl ethyl butyrate; cinnamyl acetate; 2-phenoxyethyl isobutyrate; 4-methoxybenzyl acetate;
of araliphatic ethers such as. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1 ,3-dioxane; 4,4a,5,9b-tetra-hydroindeno[1 ,2-d]-m-dioxin; 4,4a,5,9b-tetrahydro-2,4-dimethylindeno[1 ,2-d]-m-dioxin; of aromatic and araliphatic aldehydes such as. e.g., benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-isobutylphenyl)propanal; 3-(4-tert. butylphenyl)propanal; cinnamaldehyde; alpha-butylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
of aromatic and araliphatic ketones such as acetophenone; 4-methylacetophenone; 4-methoxyacetophenone; 4-tert.- butyl-2,6-dimethylaceto-phenone; 4-phenyl-2-butanone; 4-(4-hydroxyphenyl)-2-butanone; 1-(2-naphtha-lenyl)ethanone;2-benzofuranylethanone;(3-methyl-2-benzofuranyl)ethanone; benzophenone; 1 , 1 ,2,3,6-hexamethyl-5-indanyl methyl ketone; 6-tert. butyl-1 , 1-di-methyl-4-indanyl methyl ketone; 1-[2,3-dihydro-1 , 1 ,2,6-tetramethyl-3-(1-methylethyl) -1 H-5-indenyl]ethanone; 5',6',7',8'-tetrahydro-3',5',5',6',8',8'-hexamethyl-2-aceto-naphthone;
of aromatic and araliphatic carboxylic acids and their esters, e.g. Benzoic acid; phenylacetic acid; methyl benzoate; ethyl benzoate; hexyl benzoate; benzyl benzoate; methyl phenyl acetate; ethyl phenyl acetate; geranyl phenyl acetate; phenyl ethyl phenyl acetate; methyl cinnamate; ethyl cinnamate; benzyl cinnamate; phenyl ethyl cinnamate; cinnamyl cinnamate; allyl phenoxy acetate; methyl salicylate; hexyl salicylate; cyclohexyl salicylate; cis-3-hexenyl salicylate; benzyl salicylate; phenyl ethyl salicylate; methyl 2,4-dihydroxy-3,6-dimethylbenzoate; ethyl 3-phenyl glycidate; ethyl 3-methyl-3-phenyl glycidate;
of heterocyclic compounds such as 2,5-dimethyl-4-hydroxy-2H-furan-3-one ; 2-ethyl-4-hyd roxy-5-methyl-2H-furan-3-oη ; 3-hyd roxy-2-m ethyl-4H-pyran-4-one; 2-ethyl-3-hydroxy-4H-pyran-4-one;
of lactones such as. 1 ,4-octanolide; 3-methyl-1 ,4-octanolide; 1 ,4-nonanolide; 1 ,4-decanolide; 8-decen-1 ,4-olide; 1 ,4-undecanolide; 1 ,4-dodecanolide; 1 ,5-decanolide; 1 ,5-dodecanolide;4-methyl-1 ,4-decanolide; 1 , 15-pentadecanolide; 1 , 16-hexadecanolide; 9-hexadecene-1 ,16-olide; 10-oxa-1 ,16-hexadecanolide; 1 1-oxa-1 ,16-hexadecanolide; 12-oxa-1 ,16-hexadecanolide; ethylene-1 ,12-dodecanedioate; ethylene-1, 13-tridecanedioate; 2,3-dihydrocoumarin; octahydrocoumarin.

In a further preferred embodiment of the invention, at least one compound of Formula I is preferably combined with one or more, particularly preferably with two, three, four, five or more other fragrances.

Preferably, at least one compound of Formula I, in particular 1,4-dimethylcyclohept-3-ene-1-carbonitrile and/or 1,4-dimethylcyclohept-4-ene-1-carbonitrile is combined with one or more, particularly preferably with two, three, four, five or more other fragrances.

Perfume oil compositions (=fragrance mixtures) containing at least one compound of Formula I are advantageously used for perfuming in liquid form, undiluted or diluted with a solvent. Suitable solvents for this are e.g. Ethanol, isopropanol, diethylene glycol monoethyl ether, glycerin, propylene glycol, 1, 2-butylene glycol, dipropylene glycol, diethyl phthalate, triethyl citrate, isopropyimyristate etc.

Furthermore, fragrance compositions according to the invention can be adsorbed on a carrier, which ensures both a fine distribution of the fragrances in the product and a controlled release during use. Such carriers can be porous inorganic materials such as light sulfate, silica gels, zeolites, plasters, clays, clay granules, aerated concrete, etc. or organic materials such as wood, cellulose-based substances, sugar, dextrins (e.g. maltodextrin) or plastics such as PVC, polyvinyl acetates or polyurethanes. The resulting combination of compositions according to the invention and carrier substance is also to be understood as a fragrance composition according to the invention.

Fragrance compositions according to the invention can also be microencapsulated, spray-dried, as inclusion complexes or as extrusion products and added in this form to a product to be perfumed, for example.

If necessary, the properties of the compositions modified in this way can be further optimized by so-called "coating" with suitable materials with a view to a more targeted release of fragrances, for which purpose waxy plastics such as polyvinyl alcohol are preferably used. The resulting products in turn represent articles according to the invention.

A further aspect of the present invention relates to the use of the fragrance composition to modify an existing fragrance to make it rounder, more natural, more volume, clearer, fresher, stronger and/or fruitier. Preferred embodiments listed above also apply to the use of the fragrance composition.

Fragrance compositions according to the invention can advantageously be used in concentrated form, in solutions or in the modified form described above for the production of perfumed articles according to the invention, such as. B. perfume extracts, eau de perfumes, eau de toilettes, aftershave, eau de colognes, pre-shave products, splash colognes and perfumed refreshing towels and the perfuming of acidic, alkaline and neutral cleaning agents such as floor cleaners, window glass cleaners, dishwashing detergents , Bathroom and sanitary cleaners, scouring milk, solid and liquid toilet cleaners, powder and foam carpet cleaners, textile fresheners, ironing aids, liquid detergents, powder detergents, laundry pretreatment agents such as bleaches, soaking agents and stain removers, fabric softeners, laundry soaps, washing tablets, and disinfectants Air fresheners in liquid, gel-like or applied form on a solid carrier, aerosol sprays, waxes and polishes such as furniture polishes, floor waxes, shoe creams and personal care products such as solid and liquid soaps, shower gels, shampoos, shaving soaps, shaving foams s, bath oils, cosmetic emulsions of the oil-in-water, of the water-in-oil and of the water-in-oil-in-water type such as, for example, skin creams and lotions, face creams and lotions, sun protection creams and lotions, after-sun creams and lotions, hand creams and lotions, foot creams and lotions, depilatory creams and lotions, after-shave creams and lotions, tanning creams and lotions, hair care products such as hair sprays, hair gels, setting hair lotions , Hair conditioners, permanent and semi-permanent hair dyes, hair shaping agents such as cold waves and hair straighteners, hair lotions, hair creams and lotions, deodorants and antiperspirants such as underarm sprays, roll-ons, deodorant sticks, deodorant creams, products in decorative cosmetics such as eye shadow, nail varnish, Lipsticks, mascara and candles, lamp oils, incense sticks, insecticides, repellants and fuels.

Of course, fragrance compositions according to the invention can also be included in cosmetic compositions or household compositions.

Accordingly, another aspect of the present invention relates to a perfumed product. A perfumed product according to the invention comprises at least one compound of Formula I 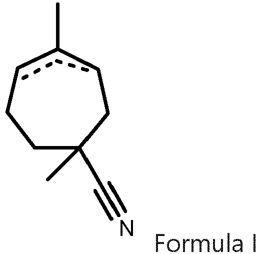 wherein each of the dotted lines represents a carbon-carbon single bond or a carbon-carbon double bond.

Preferred perfumed products according to the invention are detergents and cleaning products, hygiene or care products, especially products from the area of body and hair care, cosmetics and household products.

Preferred products are for example perfume extraits, eau de parfums, eau de toilettes, aftershaves, eau de colognes, pre-shave products, splash colognes and perfumed refreshing tissues as well as perfumed or to be perfumed acidic, alkaline and neutral detergents, such as e.g. floor cleaners, window glass cleaners, dishwashing detergents, bathroom and sanitary cleaners, scouring agent, solid or liquid toilet cleaners, toilet sticks, toilet stones (liquid or solid), powdery or foamy carpet cleaners, liquid detergents, powdery detergents, laundry pretreatment agents such as bleaches, soaking agents and stain removers, fabric softeners, laundry soaps, laundry tablets, disinfectants, surface disinfectants as well as air improvers in liquid or gel-like form or applied to a solid carrier, particularly for deodorization of exhaust air from air conditioning and industrial processes, as well as air improvers in the form of aerosol or pump sprays, waxes and polishes such as furniture polishes, floor waxes, shoe creams, strengthening, impregnating or deodorizing textile treatment agents, diapers, sanitary towels, panty liners, plasters, as well as personal care agents such as e.g. solid and liquid soaps, shower gels, shampoos, shaving soap, shaving foams, bathing oils, damp cleaning cloths, cosmetic emulsion of the oil-in-water, water-in-oil and water-in-oil-in-water type such as e.g. skin creams and lotions, face creams and lotions, sun protection creams and lotions, after sun creams and lotions, hand creams and lotions, foot creams and lotions, depilatory creams and lotions, aftershave creams and lotions, tanning creams and lotions, hair care products such as e.g. hair sprays, hair gels, strengthening hair lotions, hair conditioners, permanent or semi-permanent hair dyes, hair forming agents such as cold waves and hair smoothing agents, hair tonics, hair creams and lotions, deodorants and antiper- spirants such as e.g. armpit sprays, roll-ons, deo sticks, deo creams, products for decorative cosmetic such as e.g. eyeshadow, makeups, lipsticks, mascara as well as candles, lamp oils, incense sticks, animal litter, cat litter, insecticides, repellents, liquid and gaseous fuels, heating oils and heating gases.

Particularly preferred perfumed products according to the invention are selected from the group consisting of perfume extraits, eau de perfumes, eau de toilettes, aftershaves, eau de colognes, pre-shave products, splash colognes, perfumed refreshing tissues, acidic, alkaline and neutral detergents, textile fresheners, ironing aids, liquid detergents, powdery detergents, laundry pretreatment agents, fabric softeners, laundry soaps, laundry tablets, disinfectants, surface disinfectants, air improvers, aerosol sprays, waxes and polishes, per- sonal care agents, hand creams and lotions, foot creams and lotions, depilatory creams and lotions, aftershave creams and lotions, tanning creams and lotions, hair care products, deodorants and antiperspirants, products for decorative cosmetic, candles, lamp oils, incense sticks, insecticides, repellents and fuels.

For purposes of clarification it has to be mentioned that (perfumed) products according to the invention within the scope of the present text are to be understood as products that have been caused or produced on purpose, but not as naturally occurring substance mixtures, for example such as the ones that can be obtained from plant-based starting materials by means of extraction.

The compounds according to the present invention are preferably a constituent of a per-fume composition according to the invention. Therefore, in a preferred embodiment, a perfumed product comprises a perfume composition according to the invention, and a carrier or a substrate, wherein the carrier or the substrate is in direct contact with said perfume composition. The substrate is for example a solid substrate or the carrier is for example a solid carrier. The carrier or substrate guarantees a fine distribution of the perfume compo- sition inside the product as well as a controlled release during application. Such carriers may be porous inorganic materials such as silica gels, zeolites, gypsums, clay, clay granules, aerated concrete etc. or organic materials such as woods and cellulose-based substances.

The compounds according to the present invention to be used according to the invention or perfume compositions according to the present invention may also be present in micro- encapsulated or spray-dried form, as inclusion complexes or as extrusion products and can be added to a product in this form.

If applicable, the properties of compounds according to the present invention to be used according to the invention or of perfume compositions according to the present invention can be further optimized by means of so-called"coating" with suitable materials with regard to a more targeted release, wherein preferably wax-like plastic materials such as e.g. polyvinyl alcohol are used.

A microencapsulation of the compounds according to the present invention to be used according to the invention or perfume compositions according to the present invention can take place, for example, by means of the so-called coacervation process with the aid of capsule materials, e.g. of polyurethane-like substances or soft gelatin. Spray-dried compounds according to the present invention can be produced, for example, by means of spray-drying of a compound according to the invention, i.e. of an emulsion or dispersion containing a compound according to the present invention or a perfume compositions according to the present invention, wherein modified starches, proteins, dextrins and/or plant- based gums can be used as carrier substance. Inclusion complexes can be produced, for example, by means of addition of dispersions, which are or comprise a compound according to the present invention or a perfume compositions according to the present invention, and cyclodextrins or urea derivatives into a suitable solvent, e.g. water. Extrusion products can take place by means of fusion of a compound according to the present invention or a perfume compositions according to the present invention with a suitable wax-like substance and extrusion with subsequent solidification, in a suitable solvent, e.g. isopropanol, if applicable.

The compounds according to the present invention or fragrance compositions according to the present invention can be used in many preparations or products, wherein they are preferably combined with one or several of the following excipients or active ingredients: Preserving agents, abrasives, anti-acne agents, agents against skin aging, antibacterial agents, anticellulite agents, antidandruff agents, antiinflammatory agents, irritation preventing agents, irritation inhibiting agents, antimicrobial agents, antioxidants, astringents, sweat inhibiting agents, antiseptic agents, antistatic agents, binders, buffers, carrier materials, chelate builders, cell stimulants, cleaning agents, caring agents, depilatory agents, surface active agents, deodorizing agents, antiperspirants, plasticizers, emulsifiers, enzymes, ethereal oils, fibres, fixators, foam builders, foam stabilizers, substances to prevent foaming, foam boosters, fungicides, gelatinizing agents, gelforming agents, hair care products, hair forming products, smoothing agents, moisturizing agents, dampening substances, moist-keeping substances, bleaching agents, (textile- strengthening agents, stain removing agents, optical brightening agents, impregnating agents, dirt-repellent agents, friction-lowering agents, lubricants, moisturizing creams, ointments, opacifiers, plasticizing agents, covering agents, polish, glazing agents, polymers, powders, proteins, regreasing substances, abrasive agents, silicones, skin soothing agents, skin cleaning agents, skin caring agents, skin healing agents, skin lightening agents, skin protecting agents, skin sof- tening agents, cooling agents, skin cooling agents, warming agents, skin warming agents, stabilizers, UV-absorbing agents, UV-filters, detergents, fabric softeners, suspending agents, skin tanning agents, thickening agents, vitamins, oils, waxes, fats, phospholipids, saturated fatty acids, mono- or polyunsaturated fatty acids, ohydroxy acids, polyhydroxy fatty acids, liquefying agents, dyes, color-protecting agents, pigments, anticorrosives, aromas, flavorings, aromatic substances, polyols, surfactants, electrolytes, organic solvents or silicon derivatives.

According to one embodiment of the present invention a preferred product according to the invention, particularly a deodorant or the like, additionally contains (depending on the desired mode of action) one or several of the following active substances:
(1 ) antimicrobially active substances that inhibit the development of microorganisms that are responsible for the smell of perspiration; for example Triclosana (5-chloro-2-(2,4- dichlorophenoxy)phenol), triclocarban, chlorhexidine, chlorhexidine hydrochloride, chlorhexidine diacetate, chlorhexidine digluconate, 2-phenoxyethanol, farnesol, glycerin esters and -ethers such as glyceryl monolaurate, glyceryl monocaprinate, hexoxyglycerin, octoxyglycerin (= ethylhexylglycerin, 3-(2-ethylhexyloxy-1 ,2-pro- panediol) or Sensiva^{®} SC 50 (by Schiilke & Mayr), aliphatic 1 ,2-diols such as e.g. 1 ,2-decanediol (EP 1 269 983), araliphatic alcohols such as for example described in EP 799 174, preferably 4-methyl-4-phenyl-2-pentanol (Vetikol; WO 03/024907) or 2-methyl-4-phenyl-2-butanol (1 ,1-dimethyl-3-phenylpropanol, alpha, alpha-dime- thylphenethylcarbinol), I-menthyl methyl ether as described in WO 02/41861 , 2-ben- zylheptan-1-ol (Jasmol; 2-n-pentyl-3-phenylpropan-1-ol), 2,2-dimethyl-3-phenylpro- panol (muguet alcohol; cf. US 4,091 ,090), antimicrobially active secondary alcohols, such as for example described in WO 2005/004601 , particularly 3-methyl-6-phenyl- 2-hexanol, 4-(2,4-dimethylphenyl)-2-butanol, 6-(4-isopropylphenyl)-3-methyl-2-hex- anol, 4-(2,4,5-trimethylphenyl)-2-butanol, 3,3-dimethyl-4-phenyl-2-butanol, 3-me- thyl-4-(2-methylphenyl)-2-butanol, 6-(3,4-dimethylphenyl)-2-hexanol, aliphatic carboxylic acids such as 2-hexyloctanoic acid, 2-hexyldecanoic acid, 2-butyloctanoic acid or 2-butyldecanoic acid;
(2) enzyme inhibiting substances that inhibit the effect of enzymes that participate in the formation of smell of perspiration; for example, citric acid esters and metal-chelating substances such as EDTA (ethylenediaminetetraacetic acid), EGTA [ethylenebis(ox- yethylenenitrilo)-tetraacetic acid] and DTPA (diethylenetriaminepentaacetic acid, pentetic acid);
(3) odor absorbing substances that absorb substances that are responsible for the smell of perspiration; for example, zinc rizinoleate, cyclodextrins; (4) antiperspirants that inhibit sweat secretion and thus eliminate the breeding grounds of bacteria that are responsible for body odor. Astringent metal salts are generally preferably used as antiperspirants, particularly inorganic and organic metal salts of the elements aluminium, zinc, magnesium, tin and zircon as well as their mixtures, wherein particularly halogenides such as aluminium chloride, alkaline aluminium hydroxychlorides, zirconyl oxychlorides and zirconyl hydroxychlorides as well as their mixtures are used. Often these aluminium and zirconium salts and mixtures thereof are also used in a complexed form, wherein as complex builders preferably propylene glycol, polyethylene glycol or glycine are used. The present invention also relates to a method for producing a perfumed product, particularly a perfumed product according to the invention, comprising the following steps: i) Providing a compound according to the present invention or a perfume composition according to the present invention, ii) providing one or several further components of the perfumed product to be produced, and iii) contacting or mixing the further components provided in step ii) with a sensorially effective amount of the components provided in step i).

It is also preferred that the total amount of compounds of Formula I, based on the total weight of the perfumed product, is in the range from 0.00001 to 10% by weight, preferably from 0.0001 to 5% by weight %, particularly preferably 0.001 to 2% by weight, more preferably 0.005 to 1% by weight.

As mentioned above, the compounds of Formula I are preferably selected from 1,4-dimethylcyclohept-3-ene-1-carbonitrile according to Formula II and/or 1,4-dimethylcyclohept-4-ene-1-carbonitrile according to Formula III.

Further preferred embodiments listed above also apply to perfumed products of the present invention.

Another aspect of the invention relates to a method for modifying an existing fragrance to make it rounder, more natural, more volume, clearer, fresher, stronger and/or fruitier comprising or consisting of the following steps:
(a) providing at least one compound of Formula I 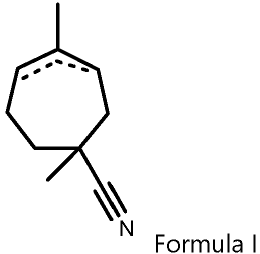 wherein each of the dotted lines represents a carbon-carbon single bond or a carbon-carbon double bond,
(b) providing at least one further fragrance,
(c) adding at least one compound of Formula I to the at least one further fragrance in a sensory effective amount sufficient to modify an existing fragrance to make it rounder, more natural, more volume, clearer, fresher, stronger and/or fruitier.

Preferably, the weight ratio of the at least one compound of Formula I to the total amount of other fragrances is in the range of 1:10 000 to 1:10, preferably from 1:1000 to 1:100.

In this context, it is preferred if the total amount the at least one compound of Formula I is in the range from 0.0001 to 99.9% by weight, preferably from 0.001 to 99.5% by weight, particularly preferably from 0.01 to 99% by weight, from 0.01 to 90% by weight, from 0.01 to 50% by weight, from 0.01 to 20% by weight and particularly preferably from 0.01 to 10% by weight, in particular from 0.05 to 1% by weight, in each case based on the total weight of the composition.

Preferably, at least one compound of Formula I is combined with one or more, particularly preferably with two, three, four, five or more other fragrances.

Further preferred embodiments listed above also apply to method of the present invention.

A further aspect of the invention relates to a method for fragrancing a compound or composition, comprising the step of applying at least one compound of Formula I 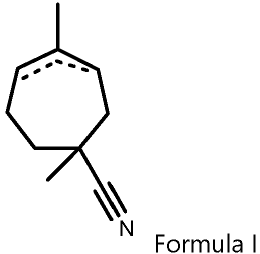
wherein each of the dotted lines represents a carbon-carbon single bond or a carbon-carbon double bond,
to that compound or composition.

Further preferred embodiments listed above also apply to method of the present invention.

Another problem to be solved by the present invention was to provide novel methods for the production of the compounds according to the present invention.

In one embodiment according to the invention there is provided a method for the preparation of a compound according to Formula I, 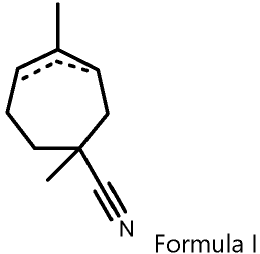
wherein each of the dotted lines represents a carbon-carbon single bond or a carbon-carbon double bond, comprising the step of
   - reacting 1,5-dimethlybicyclo [3.2.1] octan-8-one oxime according to Formula IV 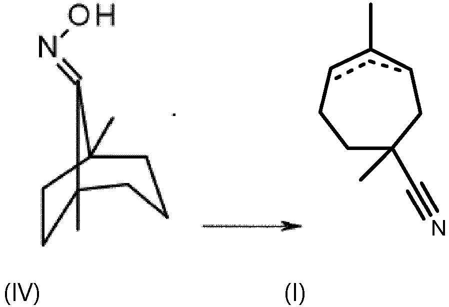
to get at least one compound of Formula I.

Preferably, the compounds according to Formula I are selected from 1,4-dimethylcyclohept-3-ene-1-carbonitrile according to Formula II and/or 1,4-dimethylcyclohept-4-ene-1-carbonitrile according to Formula III.

In a preferred embodiment according to the invention, the method comprises one or more of the following steps:
(a) providing 1,5-dimethlybicyclo [3.2.1] octan-8-one oxime according to Formula IV;
(b) mixing 1,5-dimethlybicyclo [3.2.1] octan-8-one oxime according to Formula IV with tolune;
(c) heating the mixture obtained in step (b);
(d) adding a mixture of an acid in tolune;
(e) heating the mixture obtained in step (d) refluxed via azeotropic distillation for 3-5 h;
(f) purifying the obtained product.

Preferably, the acid of step (d) is phosphoric acid. Further preferred embodiments listed above also apply to method of the present invention.

In the following, the invention is further characterized on the basis of the examples. Unless otherwise stated, all specifications thereby relate to the weight.

### Example 1: Production method of 1,4-dimethylcyclohept-3-ene-1-carbonitrile and 1,4-dimethylcyclohept-4-ene-1-carbonitrile.

A mixture of 1,5-dimethlybicyclo [3.2.1] octan-8-one oxime (250.87 g, 1500 mmol) in tolune (800 mL) was heated to 100 °C, to this very slowly a mixture of phosphoric acid (108.6 gm, 942 mmol) in tolune (200 mL) was added. After the addition, the mixture was heated refluxed via azeotropic distillation for 3-5 h. After competion of reaction, the organic layer was washed with sat. NaHCO₃, water and evaoprated to get crude product (249.0 g), which was further purified by distillation (Bt=85-95; Ht=42-45 at 1.1 mbar) to get title compound with 3:2 of 1,4-dimethylcyclohept-4-ene-1-carbonitrile : 1,4-dimethylcyclohept-3-ene-1-carbonitrile (194.0 g, 87%)
MS: 149, 134, 122, 107, 93, 82, 67, 53, 41, 27.
¹H NMR (400 MHz, CDCl₃) δ 5.53 (ddq, *J* = 8.3, 5.1, 1.7 Hz, 1H), 5.49 - 5.42 (m, 1H), 2.54 - 2.43 (m, 1H), 2.37 - 2.30 (m, 1H), 2.30 - 2.22 (m, 1H), 2.22 - 2.11 (m, 2H), 2.11 - 2.04 (m, 2H), 2.04 - 1.96 (m, 1H), 1.97 - 1.85 (m, 2H), 1.78 (d, *J* = 1.7 Hz, 3H), 1.72 (d, *J* = 1.8 Hz, 3H), 1.69 - 1.58 (m, 3H), 1.48 - 1.32 (m, 3H), 1.37 (s, 3H), 1.35 (s, 3H).
¹³C NMR (101 MHz, CDCl₃) δ 144.51, 140.03, 125.01, 124.81, 124.44, 119.82, 43.48, 39.14, 37.87, 37.64, 36.57, 35.02, 33.55, 29.69, 228.16, 27.94, 25.90, 25.88, 23.88, 22.76.
Odor: coconut, green, fatty.

## Claims

1. At least one compound of Formula I 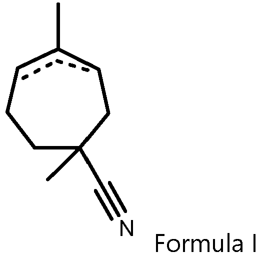 wherein each of the dotted lines represents a carbon-carbon single bond or a carbon-carbon double bond.

2. The at least one compound of Formula I, wherein the compound is 1,4-dimethylcyclohept-3-ene-1-carbonitrile

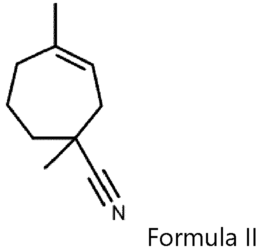

3. The at least one compound of Formula I, wherein the compound is 1,4-dimethylcyclohept-4-ene-1-carbonitrile

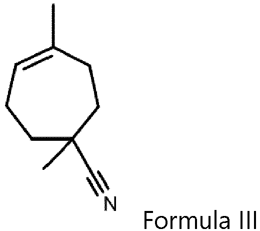

4. Mixture, comprising or consisting of at least two compounds according to Formula I.

5. Mixture according to claim 4, wherein the at least two compounds are selected from
(a) 4-dimethylcyclohept-3-ene-1-carbonitrile 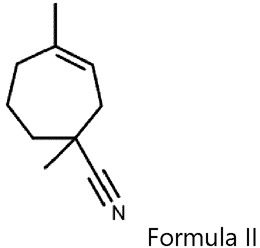 and
(b) 1,4-dimethylcyclohept-4-ene-1-carbonitrile

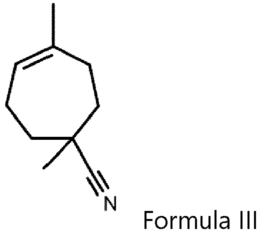

6. Use of a compound according to Formula I as a fragrance, 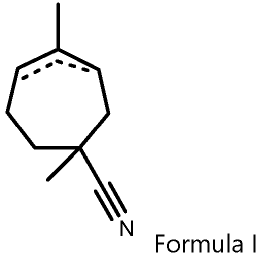 wherein each of the dotted lines represents a carbon-carbon single bond or a carbon-carbon double bond.

7. Use according to claim 7, wherein the compound is selected from 1,4-dimethylcyclohept-3-ene-1-carbonitrile according to Formula II and/or 1,4-dimethylcyclohept-4-ene-1-carbonitrile according to Formula III.

8. Use according to claim 6 and/or 7 in fragrance compositions to obtain at least one of the following fragrance notes: coconut and green.

9. Fragrance composition comprising or consisting of at least one compound of Formula I 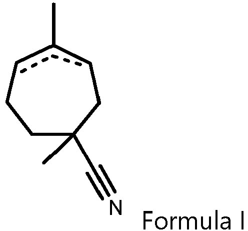
wherein each of the dotted lines represents a carbon-carbon single bond or a carbon-carbon double bond,
and at least one further fragrance which is no compound of Formula I.

10. Fragrance composition according to claim 9, wherein the weight ratio of the at least one compound of Formula I to the total amount of other fragrances is in the range of 1:1000 to 1:0.1.

11. Fragrance composition according to claim 9 and/or 10, wherein the amount of the at least one compound of Formula I is in the range of 0.01 to 50% by weight, based on the total weight of the fragrance composition.

12. Fragrance composition according to at least one of claims 9 to 11, wherein the amount of the at least one compound of Formula I is in the range of 0.01 to 10% by weight, based on the total weight of the fragrance composition.

13. Perfumed product comprising at least one compound of Formula I 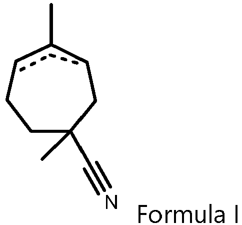 wherein each of the dotted lines represents a carbon-carbon single bond or a carbon-carbon double bond.

14. Perfumed product according to claim 13, **characterized in that** the amount of compound of formula (II) as defined in claim 2 and/or the compound of formula (III) as defined in claim 3 is in the range of 0.0001 to 5% by weight, preferably in the range from 0.001 to 2.5% by weight, in each case based on the total weight of the product.

15. Method for the preparation of a compound according to Formula I, 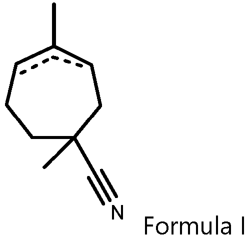
wherein each of the dotted lines represents a carbon-carbon single bond or a carbon-carbon double bond, comprising the step of
- reacting 1,5-dimethlybicyclo [3.2.1] octan-8-one oxime according to Formula IV 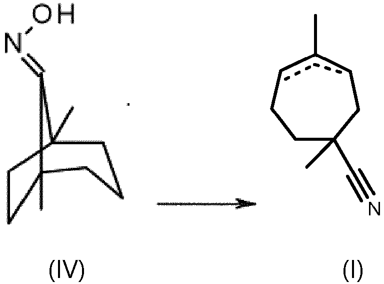
to get at least one compound of Formula I.

## Patentansprüche

1. Mindestens eine Verbindung der Formel I, 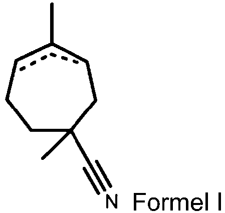 wobei jede der gestrichelten Linien eine Kohlenstoff-Kohlenstoff-Einfachbindung oder eine Kohlenstoff-Kohlenstoff-Doppelbindung darstellt.

2. Die mindestens eine Verbindung der Formel I, wobei es sich bei der Verbindung um 1,4-Dimethylcyclohept-3-en-1-carbonitril handelt.

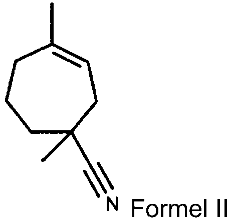

3. Die mindestens eine Verbindung der Formel I, wobei es sich bei der Verbindung um 1,4-Dimethylcyclohept-4-en-1-carbonitril handelt.

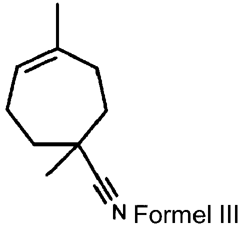

4. Gemisch, umfassend oder bestehend aus mindestens zwei Verbindungen gemäß Formel I.

5. Gemisch gemäß Anspruch 4, wobei die mindestens zwei Verbindungen ausgewählt sind aus
(a) 4-Dimethylcyclohept-3-en-1-carbonitril 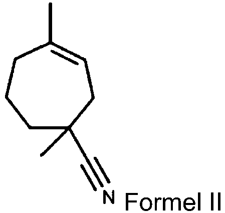 und
(b) 1,4-Dimethylcyclohept-4-en-1-carbonitril.

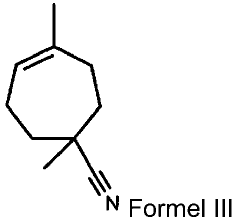

6. Verwendung einer Verbindung gemäß Formel I als Duftstoff, 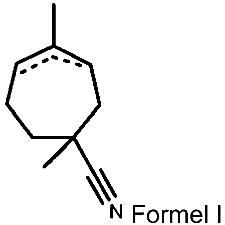 wobei jede der gestrichelten Linien eine Kohlenstoff-Kohlenstoff-Einfachbindung oder eine Kohlenstoff-Kohlenstoff-Doppelbindung darstellt.

7. Verwendung gemäß Anspruch 7, wobei die Verbindung ausgewählt ist aus 1,4-Dimethylcyclohept-3-en-1-carbonitril gemäß Formel II und/oder 1,4-Dimethylcyclohept-4-en-1-carbonitril gemäß Formel III.

8. Verwendung gemäß Anspruch 6 und/oder 7 in Duftkompositionen, um mindestens eine der folgenden Duftnoten zu erzielen: Kokosnuss und Grün.

9. Duftzusammensetzung, umfassend oder bestehend aus mindestens einer Verbindung der Formel I, 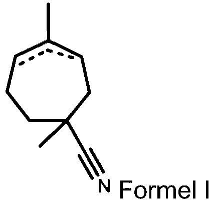
wobei jede der gestrichelten Linien eine Kohlenstoff-Kohlenstoff-Einfachbindung oder eine Kohlenstoff-Kohlenstoff-Doppelbindung darstellt,
und mindestens einem weiteren Duftstoff, der keine Verbindung der Formel I ist.

10. Duftzusammensetzung gemäß Anspruch 9, wobei das Gewichtsverhältnis der mindestens einen Komponente der Formel I zur Gesamtmenge der übrigen Duftstoffe im Bereich von 1:1000 bis 1:0,1 liegt.

11. Duftzusammensetzung gemäß Anspruch 9 und/oder 10, wobei die Menge der mindestens einen Komponente der Formel I im Bereich von 0,01 bis 50 Gew.-%, bezogen auf das Gesamtgewicht der Duftzusammensetzung, liegt.

12. Duftzusammensetzung gemäß mindestens einem der Ansprüche 9 bis 11, wobei die Menge der mindestens einen Verbindung der Formel I im Bereich von 0,01 bis 10 Gew.-%, bezogen auf das Gesamtgewicht der Duftzusammensetzung, liegt.

13. Parfümiertes Produkt, das mindestens eine Verbindung der Formel I umfasst, 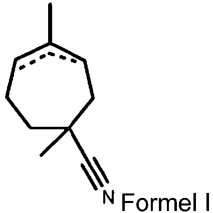 wobei jede der gestrichelten Linien eine Kohlenstoff-Kohlenstoff-Einfachbindung oder eine Kohlenstoff-Kohlenstoff-Doppelbindung darstellt.

14. Parfümiertes Produkt nach Anspruch 13, **dadurch gekennzeichnet, dass** die Menge der Verbindung der Formel (II) nach Anspruch 2 und/oder der Verbindung der Formel (III) nach Anspruch 3 im Bereich von 0,0001 bis 5 Gew.-%, vorzugsweise im Bereich von 0,001 bis 2,5 Gew.-%, jeweils bezogen auf das Gesamtgewicht des Produkts, liegt.

15. Verfahren zur Herstellung einer Verbindung nach Formel I, 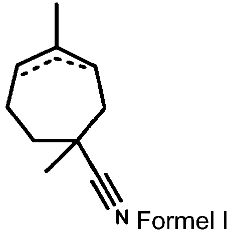
wobei jede der gestrichelten Linien eine Kohlenstoff-Kohlenstoff-Einfachbindung oder eine Kohlenstoff-Kohlenstoff-Doppelbindung darstellt, umfassend den Schritt
- Umsetzen von 1,5-Dimethylbicyclo-[3.2.1]-octan-8-onoxim gemäß Formel IV, 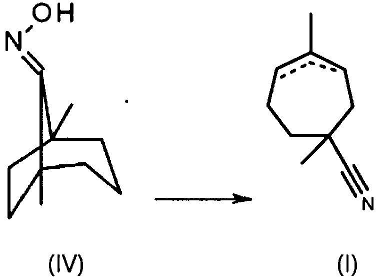
um mindestens eine Verbindung der Formel I zu erhalten.

## Revendications

1. Au moins un composé de formule I 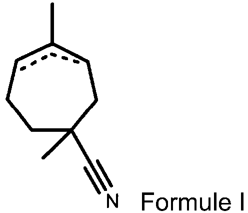 où chacune des lignes en pointillé représente une liaison simple carbone-carbone ou une liaison double carbone-carbone.

2. L'au moins un composé de formule I, dans lequel le composé est le 1,4-diméthylcyclohept-3-ène-1-carbonitrile

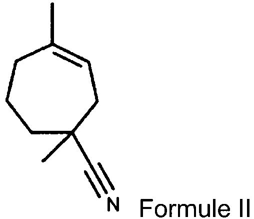

3. L'au moins un composé de formule I, dans lequel le composé est le 1,4-diméthylcyclohept-4-ène-1-carbonitrile

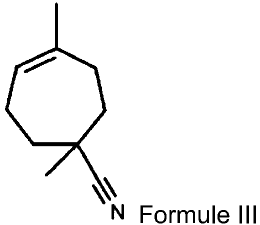

4. Mélange comprenant ou consistant en au moins deux composés selon la formule I.

5. Mélange selon la revendication 4, dans lequel les au moins deux composés sont choisis parmi
(a) le 4-diméthylcyclohept-3-ène-1-carbonitrile 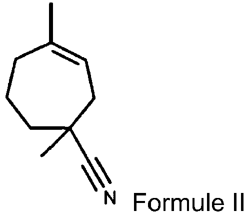 et
(b) le 1,4-diméthylcyclohept-4-ène-1-carbonitrile

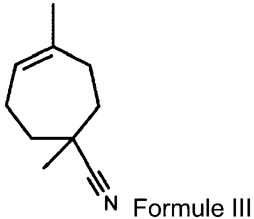

6. Utilisation d'un composé selon la formule I comme parfum, 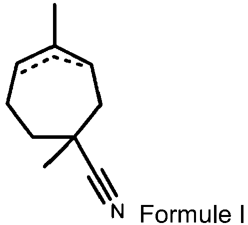 où chacune des lignes en pointillé représente une liaison simple carbone-carbone ou une liaison double carbone-carbone.

7. Utilisation selon la revendication 7, dans laquelle le composé est choisi parmi le 1,4-diméthylcyclohept-3-ène-1-carbonitrile selon la formule Il et/ou le 1,4-diméthylcyclohept-4-ène-1-carbonitrile selon la formule III.

8. Utilisation selon la revendication 6 et/ou 7 dans des compositions de parfum pour obtenir au moins l'une des notes de parfum suivantes : noix de coco et verte.

9. Composition de parfum comprenant ou consistant en au moins un composé de formule I 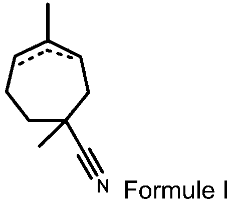
où chacune des lignes en pointillé représente une liaison simple carbone-carbone ou une liaison double carbone-carbone,
et au moins un parfum supplémentaire qui n'est pas un composé de formule I.

10. Composition de parfum selon la revendication 9, dans laquelle le rapport en poids de l'au moins un composé de Formule I à la quantité totale des autres parfums est dans la plage de 1:1000 à 1:0,1.

11. Composition de parfum selon la revendication 9 et/ou 10, dans laquelle la quantité de l'au moins un composé de formule I est dans la plage de 0,01 à 50 % en poids, par rapport au poids total de la composition de parfum.

12. Composition de parfum selon au moins l'une des revendications 9 à 11, dans laquelle la quantité de l'au moins un composé de formule I est dans la plage de 0,01 à 10 % en poids, par rapport au poids total de la composition de parfum.

13. Produit parfumé comprenant au moins un composé de formule I 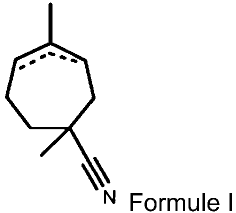 où chacune des lignes en pointillé représente une liaison simple carbone-carbone ou une liaison double carbone-carbone.

14. Produit parfumé selon la revendication 13, **caractérisé en ce que** la quantité de composé de formule (II) selon la revendication 2 et/ou de composé de formule (III) selon la revendication 3 est dans la plage de 0,0001 à 5 % en poids, de préférence dans la plage de 0,001 à 2,5 % en poids, dans chaque cas par rapport au poids total du produit.

15. Procédé de préparation d'un composé selon la formule I, 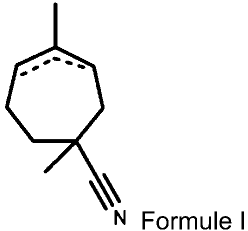
où chacune des lignes en pointillé représente une liaison simple carbone-carbone ou une liaison double carbone-carbone, comprenant l'étape consistant à
- faire réagir de l'oxime de 1,5-diméthylbicyclo [3.2.1] octan-8-one selon la formule IV 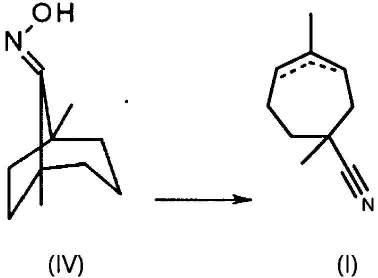
pour obtenir au moins un composé de formule I.
